# EUROPEAN PATENT APPLICATION

(11) **EP 4 572 159 A2**
(43) Date of publication of application: **18.06.2025**
(21) Application number: 25154453.2
(22) Date of filing: 06.02.2022
(51) Int. Cl.: H04B 5/00

(54) **COUPLING ELECTRICAL SIGNALS ACROSS AN ELECTRICALLY INSULATING BARRIER**

(30) Priority: 11.02.2021 EP 21156506
(62) Divisional of application: 22703667.0
(71) Applicant: Koninklijke Philips N.V., 5656 AG Eindhoven (NL)
(72) Inventor: KAHLMAN, Josephus Arnoldus Johannes Maria, Eindhoven (NL); IMMINK, Albert Hendrik Jan, Eindhoven (NL)
(74) Representative: Philips Intellectual Property & Standards

(57) **Abstract**

A system for coupling electrical signals between an interventional device (110) and a control unit (120) through an electrically insulating barrier (130), is disclosed. The system includes a connector (140). A device connector portion (140') of the connector (140) includes at least one transmitter (160) that transmits electrical signals representing i) transducer signals and ii) synchronisation signals for the transducer signals. The transducer signals are generated at a distal portion (110") of the interventional device (110), and the synchronisation signals are generated at a distal portion (110") of the interventional device or in the device connector portion (140'). A control unit connector portion (140") of the connector (140), or the control unit (120), receives the electrical signals, extracts the transducer signals and the synchronisation signals from the electrical signals, and performs a timing correction on the transducer signals using the synchronisation signals.

## Description

### Technical Field

The present disclosure relates to a system for coupling electrical signals between an interventional device and a control unit through an electrically insulating barrier. The system finds application in the medical field in general.

### Background

Interventional devices such as intravascular ultrasound "IVUS" imaging catheters, and functional measurement catheters, are routinely used to perform medical procedures on patients. Such devices may include sensors, actuators, and energy delivery devices, and are typically coupled to a control unit which generates and/or processes electrical signals associated with the interventional device. Often, a connector is disposed between the interventional device and the control unit in order that the interventional device may be coupled and de-coupled from the control unit.

Interventional procedures are typically performed by arranging a surgical drape over the patient with an opening over the insertion point at which the interventional device enters the body. The surgical drape typically covers the patient, and the patient bed, forming a barrier between a sterile zone above the drape, and a non-sterile zone below the drape.

In such arrangements it is important that portions of the interventional device that may be touched by medical practitioners during a medical procedure, remain sterile. In this respect, the act of coupling the sterile connector of the interventional device, to the control unit can be challenging because the control unit, and more particularly the mating portion of the connector that is attached to the control unit, are typically located in the non-sterile zone. Consequently, this act of coupling the sterile connector of the interventional device, to the control unit, is typically performed by a non-sterile nurse, and presents workflow challenges.

A document WO 2020/193720 A1 relates to a connector providing a connection through a flexible barrier. The connector comprises at least a device pad and at least a landing pad. The device pad is arranged in close proximity to the landing pad for contactless connection through the flexible barrier to transmit and/or receive data and/or power between each other. The device pad and the landing pad are configured to attach and/or align to each other.

However, there remains room to improve the coupling of electrical signals between an interventional device and a control unit through an electrically insulating barrier such as a surgical drape.

### Summary

According to one aspect of the present disclosure, a system for coupling electrical signals between an interventional device and a control unit through an electrically insulating barrier, is provided. The system includes a connector, comprising a device connector portion and a control unit connector portion. The device connector portion is configured to be electrically coupled to a proximal portion of the interventional device. The control unit connector portion is configured to be electrically coupled to the control unit. The device connector portion, and the control unit connector portion are configured to couple to each other across the electrically insulating barrier. The device connector portion comprises at least one transmitter, configured to transmit electrical signals representing i) transducer signals and ii) synchronisation signals for the transducer signals, the transducer signals being generated at a distal portion of the interventional device (e.g. measurement signals from a sensor/transducer, which measurement may represent at least one of an ultrasound reflection from anatomical structure, flow velocity of body fluid, pressure of body fluid), and the synchronisation signals being generated at a distal portion of the interventional device (e.g. at an application-specific integrated circuit ASIC) or in the device connector portion. The control unit connector portion comprises at least one receiver configured to receive the electrical signals when the control unit connector portion and the device connector portion are coupled to each other across the electrically insulating barrier. The control unit connector portion, or the control unit, is configured to receive the electrical signals, and to extract the transducer signals and the synchronisation signals from the electrical signals, and to perform a timing correction on the transducer signals based on the synchronisation signals. The distal portion of the interventional device may be distal end in some embodiments and the proximal portion of the interventional device may be proximal end in some further additional or alternative embodiments.

According to a further aspect, a method corresponding to the operation of the system is provided. The method, of coupling electrical signals between an interventional device and a control unit through an electrically insulating barrier, comprises:
electrically coupling a device connector portion of a connector to a proximal portion of the interventional device;
electrically coupling a control unit connector portion of the connector to the control unit;
coupling the device connector portion and the control unit connector portion to each other across the electrically insulating barrier;
transmitting electrical signals from the device connector portion and receiving the electrical signals at the control unit connector portion, wherein the electrical signals represent i) transducer signals and ii) synchronisation signals for the transducer signals, the transducer signals being generated at a distal portion of the interventional device, and the synchronisation signals being generated at a distal portion of the interventional device or in the device connector portion;
extracting the transducer signals and the synchronisation signals from the electrical signals at the control unit connector portion or at the control unit, and
performing a timing correction on the transducer signals based on the synchronisation signals.

Any system embodiment may have corresponding method embodiment, which is herewith contemplated, however for sake of conciseness the method embodiments are not further detailed separately, as they would in many instances lead to repetition of passages in the description.

Further aspects, features, and advantages of the present disclosure will become apparent from the following description of examples, which is made with reference to the accompanying drawings.

### Brief Description of the Drawings

Fig. 1 is a schematic diagram of an example of an arrangement including an interventional device 110, an electrically insulating barrier 130, and a connector 140 for coupling electrical signals between the interventional device 110 and a control unit 120 through the electrically insulating barrier 130, in accordance with some aspects of the disclosure.
Fig. 2 is a schematic diagram of a first example of a system including a connector 140 for coupling electrical signals between an interventional device 110 and a control unit 120 through an electrically insulating barrier 130, in accordance with some aspects of the disclosure.
Fig. 3 is a schematic diagram of a second example of a system including a connector 140 for coupling electrical signals between an interventional device 110 and a control unit 120 through an electrically insulating barrier 130, in accordance with some aspects of the disclosure.
Fig. 4 is a schematic diagram of a third example of a system including a connector 140 for coupling electrical signals between an interventional device 110 and a control unit 120 through an electrically insulating barrier 130, in accordance with some aspects of the disclosure.

### Detailed Description

Examples of the present disclosure are provided with reference to the following description and the figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity.

In the following description, reference is made to a system for coupling electrical signals between an interventional device and a control unit through an electrically insulating barrier. Reference is made to an IVUS imaging device that serves as an example of an interventional device. One example of an IVUS imaging device is the EagleEye^{®} catheter available from Philips Medical Systems, Best, The Netherlands. However, this device is only used as an example, and it is to be appreciated that the system may also be used with other types of IVUS imaging devices, and also with interventional devices in general. For example, the system may also be used with an interventional device such as an intravascular blood flow measurement device, an in-body tracking system, and so forth. An example of an intravascular blood flow measurement device is the FloWire, also available from Philips Medical Systems, Best, The Netherlands. The FloWire is a guidewire that uses Doppler ultrasound to measure blood flow within the vasculature. Alternatively or additionally to ultrasound imaging and/or intravascular blood flow measurement, the interventional device may be configured to provide pressure signals from an anatomical structure, such as a blood vessel. Embodiments wherein the same one or more transducer elements of the interventional device is/are configured to interfacing the body liquid to provide simultaneous or interlaced pressure measurement of the body liquid (e.g. blood in a vessel), ultrasound imaging and/or body liquid flow velocity measurement values are disclosed in the European Patent application no. 20187645.5, filed on July 24, 2020, which is incorporated herein by reference in its entirety. These embodiments to which is referred, benefit from the invention, wherein extracting the transducer signals and the synchronisation signals from the electrical signals is performed, and subsequently a timing correction on the transducer signals is carried out based on the synchronisation signals.

Reference is also made in this description to an electrically insulating barrier. In some examples, the electrically insulating barrier is provided by a surgical drape. Surgical drapes are used to form a sterile barrier between sterile and non-sterile zones in interventional procedures. Surgical drapes may be formed from various woven, and non-woven, materials. The non-conductive nature of materials typically used in surgical drapes typically result in the surgical drape providing an electrically insulating barrier. The materials used in such surgical drapes typically result in a flexible electrically insulating barrier. The surgical drape may for example include one or more non-woven layers formed from a polymer such as polyester, polypropylene, polyethylene. The surgical drape may additionally or alternatively include a woven material formed from one or more types of fiber such as cellulosic, non-cellulosic, synthetic or animal fibers. However, use of the system is not limited to these examples, and the use of surgical drapes that comprise other materials which present an electrically insulating barrier, is also contemplated.

It is also noted that in some examples, the device connector portion and the control unit connector portion may couple to each other in the absence of an electrically insulating barrier. Thus, the connector finds application in the coupling electrical signals between an interventional device and a control unit in general.

As mentioned above, interventional procedures are typically performed by arranging a surgical drape over the patient with an opening over the insertion point at which the interventional device enters the body. The surgical drape typically covers the patient and the patient bed, forming a barrier between a sterile zone above the drape, and a non-sterile zone below the drape. Interventional devices used in interventional procedures often include sensors, actuators, and energy delivery devices and are typically coupled to a control unit which generates and/or processes electrical signals associated with the interventional device. Often, a connector is disposed between the interventional device and the control unit in order that the interventional device may be coupled and de-coupled from the control unit. During such procedures, it is important to preserve the sterility of the sterile zone. In this respect, the act of coupling the interventional device connector portion, and which is typically located in the sterile zone, to the control unit connector portion, which is typically located in the non-sterile zone, can be challenging.

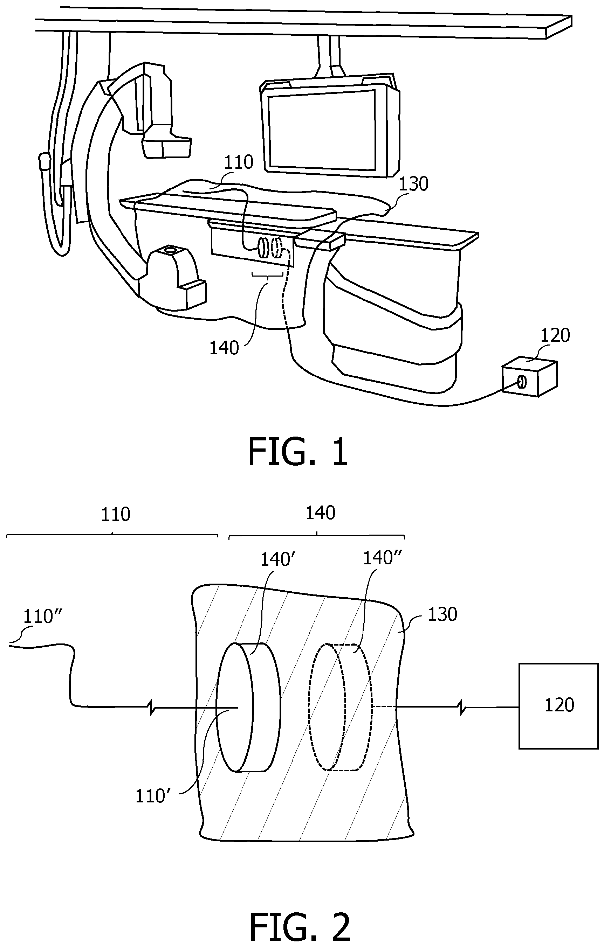

Fig. 1 is a schematic diagram of an example of an arrangement including an interventional device 110, an electrically insulating barrier 130, and a connector 140 for coupling electrical signals between the interventional device 110 and a control unit 120 through the electrically insulating barrier 130, in accordance with some aspects of the disclosure. The arrangement illustrated in Fig. 1 may be used in an interventional procedure, such as an IVUS imaging procedure. In an IVUS imaging procedure, a patient lies on the patient bed in Fig. 1, and a guidewire is inserted into their vasculature under X-ray guidance. The guidewire is inserted into the patient through a small incision in the body, and the IVUS imaging device 110 is translated over the guidewire in order to perform an IVUS imaging procedure within the vasculature. In order to comply with the stringent sterility requirements of such procedures, a surgical drape 130 is arranged over the patient. The surgical drape 130 has an opening for inserting the guidewire and the IVUS imaging device into the body. The surgical drape 130 acts as an electrically insulating barrier, and substantially covers the patient bed, including the bedrail.

In the arrangement in Fig. 1, a connector 140 is provided for coupling electrical signals between the IVUS imaging device 110 and the control unit 120 through the electrically insulating barrier 130. The control unit 120 of an IVUS imaging device is often termed a patient interface module "PIM". The PIM is typically coupled to the bedrail, or located on the patient bed next to the patient, but the PIM may alternatively be located elsewhere. In the example arrangement illustrated in Fig. 1, the control unit 120, i.e. the PIM, is located at the foot of the patient bed.

More detail on the connector 140 is provided with reference to Fig. 2, which is a schematic diagram of a first example of a system including a connector 140 for coupling electrical signals between an interventional device 110 and a control unit 120 through an electrically insulating barrier 130, in accordance with some aspects of the disclosure.

In the system illustrated in Fig. 2, the connector 140, includes a device connector portion 140' and a control unit connector portion 140''. The device connector portion 140' is configured to be electrically coupled to a proximal end 110' of the interventional device 110. The device connector portion 140' may for example include one or more electrical contacts and/or one or more electrical conductors for electrically coupling the device connector portion 140' to the proximal end 110' of the interventional device 110. The control unit connector portion 140'' is configured to be electrically coupled to the control unit 120. The control unit connector portion 140" may for example include one or more electrical contacts and/or one or more electrical conductors for electrically coupling the control unit connector portion 140'' to the control unit 120.

The device connector portion 140', and the control unit connector portion 140'' illustrated in Fig. 1 and Fig. 2 are configured to couple to each other across the electrically insulating barrier 130. In this respect, in some examples it is contemplated that the control unit connector portion 140" and the device connector portion 140' may be coupled to each other across the electrically insulating barrier 130 via magnetic force and/or via a mechanical fixture.

It is noted that the opposing surfaces of the device connector portion 140', and the control unit connector portion 140" and across which the electrical signals are transmitted, are typically electrically isolated such that there is no electrical contact between the transmitter(s) and receiver(s). Thus, there is typically no electrical contact between the electrically insulating barrier and the transmitter(s) and receiver(s), or indeed between the transmitter(s) and receiver(s) in the absence of any electrically insulating barrier. This may improve the safety and/or ability to sterilise the connector.

In some examples, the portions of the connector 140 are coupled together using magnetic force. In these examples, each of the control unit connector portion 140" and the device connector portion 140' may include a magnet having opposing polarities. In these examples, the opposing polarities of the magnets hold the control unit connector portion 140" and the device connector portion 140' in proximity to one another across the electrically insulating barrier.

In some examples, the portions of the connector 140 are coupled together using a mechanical fixture. In these examples, the control unit connector portion 140" and/or the device connector portion 140' may include a mechanical fixture such as a latch, or a friction socket, or a snap connector, and so forth, to likewise hold the control unit connector portion 140" and the device connector portion 140' in proximity to one another across the electrically insulating barrier. In some examples it is contemplated that the control unit connector portion 140" and the device connector portion 140' may be coupled to each other across the electrically insulating barrier 130 via a shape of the device connector portion 140' and a corresponding shape of the control unit connector portion 140''. For example, one portion 140', 140" of the connector may include a circular pad, and the other portion of the connector may include a corresponding circular recess, the circular pad and circular recess having dimensions such that when the pad is pressed into the recess across the electrically insulating barrier 130, the control unit connector portion 140" and the device connector portion 140' are held in proximity to one another across the electrically insulating barrier by friction. Alternative shapes to the circular shape in this example may also be used in a similar manner.

In some examples, the device connector portion 140', and the control unit connector portion 140" may couple to each other in the absence of an electrically insulating barrier. This may be also achieved using a magnetic coupling and/or a mechanical fixture, as described above. In some examples, the device connector portion 140' and the control unit connector portion 140" may couple to each other in the absence of an electrically insulating barrier, or when an electrically insulating barrier is present. This may be achieved by providing the magnetic coupling, or mechanical fixture, with sufficient tolerance to permit coupling in the absence or presence of an electrically insulating barrier.

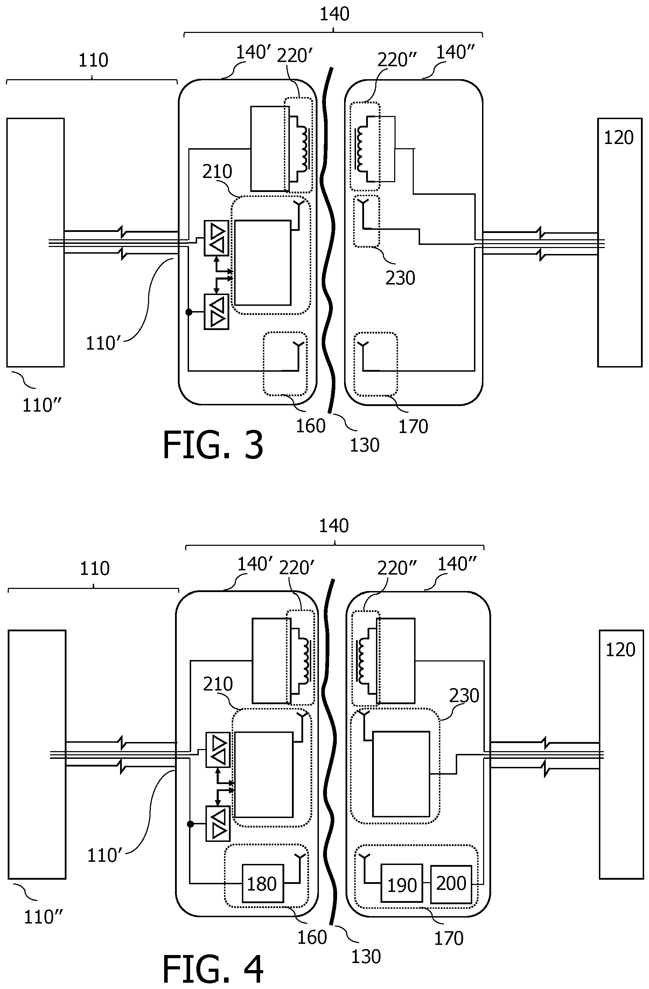

In Fig. 2, in this first example of the system, the device connector portion 140' comprises at least one transmitter (not illustrated in Fig. 2). The at least one transmitter is configured to transmit electrical signals representing i) transducer signals and ii) synchronisation signals for the transducer signals. The electrical signals are transmitted as RF signals. In general, the use of RF frequencies in the range from 5 MHz - 2000 MHz, is contemplated. In some examples, RF signals in the range from 10 MHz - 1000 MHz, is contemplated. The transducer signals are generated at a distal end 110'' of the interventional device 110, and the synchronisation signals are generated at a distal end 110" of the interventional device or in the device connector portion 140'. The control unit connector portion 140" comprises at least one receiver (not illustrated in Fig. 2) that is configured to receive the electrical signals when the control unit connector portion 140" and the device connector portion 140' are coupled to each other across the electrically insulating barrier 130. In this example, the control unit connector portion 140'', or the control unit 120, is configured to receive the electrical signals, and to extract the transducer signals and the synchronisation signals from the electrical signals, and to perform a timing correction on the transducer signals using the synchronisation signals. The transmitter and the receiver are not illustrated in Fig. 2. However, these are described below with reference to the examples in Fig. 3 and Fig. 4.

Interventional devices typically include long electrical cables between the interventional device 110 and the control unit 120. For example, an electrical cable coupling the distal end of an IVUS imaging catheter to the connector on the control unit, i.e. the patient interface module "PIM", may have a length of two metres or more. This includes a length of approximately ninety centimetres between a distal end of the IVUS imaging catheter that includes ultrasound transducers for performing IVUS imaging, and a proximal end of the IVUS imaging catheter. The proximal end of the IVUS imaging catheter remains outside the body and typically includes a Y-connector port that allows a guidewire to be inserted within the IVUS imaging catheter. An IVUS imaging catheter includes ultrasound transducers at its distal end. The ultrasound transducers generate ultrasound signals, and subsequently detect ultrasound signals, i.e. echoes from within the vasculature, in response to the generated ultrasound signals. The ultrasound signals are generated from electrical transmit pulses that are generated using a clock or sequencer circuit. In existing IVUS imaging systems, the clock or sequencer circuit is typically located in the control unit, i.e. in the PIM. The clock signals are conveyed along the more-than-two-metre length of electrical cable between the PIM and the ultrasound transducers where they are used to generate the ultrasound signals. The detected ultrasound signals, i.e. echoes, that are detected by the ultrasound transducers in response to the generated ultrasound signals, are conveyed back along the more-than-two-meters long electrical cable to the PIM as electrical signals. In the PIM, a timing correction is applied to the electrical signals representing the detected ultrasound signals. The timing correction involves applying phase delays to the ultrasound signals detected by different ultrasound transducers. The relative phase delays act to focus and/or steer the sensitivity of the ultrasound transducers to detected ultrasound signals. In so doing, the relative phase delays are used to control the generation of IVUS image data.

The inventors have determined that the electrical signal paths between IVUS imaging transducer elements and their control units, or PIMs, suffer from latency, and also fluctuating latency. Latency is also sometimes referred to as a time delay, or a phase delay. This fluctuating latency, or jitter, degrades IVUS image quality. The signal paths between other interventional devices and their control units may also be affected in a similar manner.

In the connector 140 described herein, the device connector portion 140' comprises at least one transmitter that transmits the electrical signals, and the control unit connector portion 140" comprises at least one receiver. The control unit connector portion 140'', or the control unit 120, extracts the transducer signals and the synchronisation signals from the electrical signals, and performs a timing correction on the transducer signals using the synchronisation signals. In the connector 140, since the electrical signals represent both i) the transducer signals and ii) the synchronisation signals for the transducer signals, the transducer signals and the synchronisation signals are transmitted simultaneously and over the same signal path, and so any latency, and also any fluctuations in the latency of the signal path, affects both i) the transducer signals and ii) the synchronisation signals for the transducer signals, in a similar manner. Since the synchronisation signals are used to perform a timing correction on the transducer signals, and since latency, and also any fluctuations in the latency affect both i) the transducer signals and ii) the synchronisation signals for the transducer signals, in a similar manner, the impact of latency, and latency fluctuations is reduced, and IVUS image quality is improved.

Further detail on the connector described above with reference to Fig. 1 and Fig. 2, is provided with reference to Fig. 3, which is a schematic diagram of a second example of a system including a connector 140 for coupling electrical signals between an interventional device 110 and a control unit 120 through an electrically insulating barrier 130, in accordance with some aspects of the disclosure. It is noted that the second example may include one or more features described above with reference to Fig. 1 and Fig. 2.

The system illustrated in Fig. 3 includes a connector 140 comprising a device connector portion 140' and a control unit connector portion 140''. The device connector portion 140' is configured to be electrically coupled to a proximal end 110' of the interventional device 110. The control unit connector portion 140" is configured to be electrically coupled to the control unit 120. The device connector portion 140', and the control unit connector portion 140" are configured to couple to each other across the electrically insulating barrier 130. The device connector portion 140' comprises at least one transmitter 160, configured to transmit electrical signals representing i) transducer signals and ii) synchronisation signals for the transducer signals, the transducer signals being generated at a distal end 110" of the interventional device 110, and the synchronisation signals being generated at a distal end 110" of the interventional device or in the device connector portion 140'.

The at least one transmitter 160 illustrated in Fig. 3 includes an antenna for transmitting the electrical signals representing i) transducer signals and ii) synchronisation signals for the transducer signals. The at least one transmitter 160 may also include an amplifier, not illustrated in Fig. 3, for amplifying the electrical signals.

The control unit connector portion 140" comprises at least one receiver 170 configured to receive the electrical signals when the control unit connector portion 140'' and the device connector portion 140' are coupled to each other across the electrically insulating barrier 130. The at least one receiver 170 includes an antenna for receiving the electrical signals, and may also include an amplifier, not illustrated in Fig. 3, for amplifying the received electrical signals.

In Fig. 3, the control unit 120, is configured to receive the electrical signals, and to extract the transducer signals and the synchronisation signals from the electrical signals, and to perform a timing correction on the transducer signals using the synchronisation signals. In the illustrated example, the control unit connector portion 140" may therefore include an antenna, and the electrical signals are transmitted from the antenna to the control unit via one or more conductors. In an alternative example described below with reference to Fig. 4, the control unit connector portion 140" may be configured to receive the electrical signals, and to extract the transducer signals and the synchronisation signals from the electrical signals, and to perform a timing correction on the transducer signals using the synchronisation signals. Performing this functionality in the control unit 120 as opposed to the control unit connector portion 140", permits the use of a less complex control unit connector portion 140''. To this end, the control unit 120, or the control unit connector portion 140'', may include a detector circuit such as a demodulator, or more specifically a synchronisation demodulator for extracting the transducer signals and the synchronisation signals from the electrical signals.

In some examples, the electrical signals, i.e. the combination of the transducer signals and the synchronisation signals, is frequency-modulated onto a carrier frequency, such as a RF 500 MHz carrier frequency, or another carrier frequency. In these examples the detector circuit may include an FM demodulator circuit for extracting the electrical signals, i.e. the transducer signals and the synchronisation signals, from the RF signal. In other examples, other types of modulation, and therefore other types of corresponding demodulator circuits, may be used. For example, an amplitude, phase or pulse code demodulator circuit may be used to extract the transducer signals and the synchronisation signals from the electrical signals.

In one example, the electrical signals are provided by detected IVUS imaging signals. The detected IVUS imaging signals are detected by an IVUS imaging transducer in response to the emission of ultrasound signals by the IVUS imaging transducer. The emission of ultrasound signals by the IVUS imaging transducer is achieved by a carrier signal. The carrier signal is typically in the range from 5 MHz - 50 MHz. The detected IVUS imaging signals represent ultrasound echoes within the vasculature. In this example, the transducer signals represent the ultrasound echoes, and the transducer signals are inherently modulated onto the carrier signal. In this example, the electrical signals, i.e. both i) transducer signals modulated onto the transmitted carrier signal and ii) synchronisation signals for the transducer signals, may be transmitted by the transmitter(s). These electrical signals, may be transmitted without any further modulation. However, they may optionally be further modulated to provide e.g. an AM or FM transmitted electrical signal. This further modulation has the benefit of reduced susceptibility to variations in the gap between the device connector portion 140' and the control unit connector portion 140''. The control unit 120, or the control unit connector portion 140'', may also include a timing correction circuit for performing the timing correction on the transducer signals using the synchronisation signals. The timing correction circuit may include a phase shifting circuit and/or one or more time delay circuits that are configured to provide a phase delay and/or a time delay to the transducer signals using the synchronisation signals. By way of an example, if the interventional device 110 includes an IVUS imaging device, the phase delay circuit may use the synchronisation signals to introduce relative phase delays to the transducer signals generated by the ultrasound transducers in the IVUS imaging device, and thereby focus and/or steer the sensitivity of the ultrasound transducers when detecting ultrasound signals.

In some examples, a second set of electrical signals may be transmitted between the device connector portion 140' and the control unit connector portion 140''. In these examples, the electrical signals described above comprise a first bandwidth. The second set of electrical signals have a second bandwidth that is smaller than the first bandwidth. The electrical signals, and the second set of electrical signals may be transmitted by the same transmitter 160, or the second set of electrical signals may be transmitted by another transmitter such as the second transmitter 210 illustrated in the device connector portion 140' in Fig. 3. The second set of electrical signals may represent i) a status of the interventional device and/or ii) programming data for programming a processor disposed on the device connector portion or on the interventional device and/or iii) measurement data generated by a temperature sensor or an electrocardiography sensor or a power sensor disposed on the interventional device. The effects of latency may be less important on the second set of electrical signals than the electrical signals that represent i) transducer signals and ii) synchronisation signals for the transducer signals. The second set of electrical signals may be received by the at least one receiver 170, i.e. the same receiver 170 that receives the electrical signals. Alternatively, the control unit connector portion 140" may include a second receiver 230 for receiving the second set of electrical signals. The second receiver 230, if used, includes an antenna for receiving the second set of electrical signals, and may also include an amplifier, not illustrated in Fig. 3, for amplifying the received second set of electrical signals.

In some examples, electrical power may be transmitted electromagnetically between the control unit connector portion 140" and the device connector portion 140'. In these examples, the device connector portion 140' and the control unit connector portion 140'' each include a coil 220', 220" for transmitting electrical power electromagnetically between the control unit connector portion 140" and the device connector portion 140'. The electrical power may be used to power electrical circuits in the interventional device 110 and/or in the device connector portion 140'. In these examples, the device connector portion 140' may further include at least one rectifier coupled to the coil 220' of the device connector portion 140' for rectifying electromagnetic signals received by the coil 220' from the coil 220'' of the control unit connector portion 140''. The at least one rectifier may be included in a power conditioning circuit such as a full or half-wave rectifier circuit and/or or a buck or boost converter circuit for conditioning the transmitted electrical power.

Fig. 4 is a schematic diagram of a third example of a system including a connector 140 for coupling electrical signals between an interventional device 110 and a control unit 120 through an electrically insulating barrier 130, in accordance with some aspects of the disclosure. Features identified in Fig. 4 with the same references numerals as Fig. 3 refer to corresponding features. It is noted that the third example may include one or more features described above with reference to Fig. 3. The system illustrated in Fig. 4 differs from the system illustrated in Fig. 3 in that the at least one transmitter 160 in Fig. 4 also comprises a modulator circuit 180. The modulator circuit 180 is configured to generate the electrical signals using the transducer signals and the synchronisation signals. As mentioned above in relation to the detector circuit, the modulator circuit may employ frequency modulation, amplitude modulation, pulse code modulation, or indeed another type of modulation. Thus, in some examples, the modulator circuit frequency modulates the transducer signals and the synchronisation signals onto a carrier frequency, such as a 500 MHz RF carrier frequency, or another carrier frequency, in order to generate the electrical signals. In these examples, the modulator circuit 180 may include a voltage controlled oscillator. In some examples, the synchronisation signals may be used to modulate the base frequency of the voltage controlled oscillator, and the transducer signals may be used to modulate the base frequency. In these examples the detector circuit may include an FM demodulator circuit for extracting the transducer signals and the synchronisation signals from the electrical signals. In other examples, other types of modulator circuits may be used to generate the electrical signals, such as an amplitude, phase or pulse code modulator circuit.

As mentioned above in relation to Fig. 3, in some examples, the receiver 170 in the control unit connector portion 140'' includes a detector circuit and a timing correction circuit. These are illustrated in Fig. 4 as optional features 190 and 200, respectively. These features may alternatively be disposed in the control unit 120. The detector circuit 190 is configured to extract the transducer signals and the synchronisation signals from the electrical signals. The timing correction circuit 200 is configured to perform the timing correction on the transducer signals by using the synchronisation signals to adjust a timing of the transducer signals.

**In** some examples, a clock circuit is provided for generating the synchronization signals. The clock circuit may be disposed on the interventional device 110, preferably as close as possible to the transducer, or in the device connector portion 140'. As compared to including the clock circuit in the control unit, including the clock circuit on the interventional device 110, or in the device connector portion 140' helps to further reduce the impact of latency fluctuations, and to further improve IVUS image quality. This is because the clock signals from the clock circuit travel a shorter distance along electrical conductors before they reach the ultrasound transducers where they are converted to ultrasound pulses, and are consequently less affected by phase noise or jitter, e.g. due to external interference or amplitude noise in combination with the limited bandwidth of the electrical conductors. **In** these examples, the modulator circuit 180 may be co-located with the clock circuit, or the clock circuit may be disposed on the interventional device 110, preferably as close as possible to the transducer, and the modulator circuit 180 may be disposed in the device connector portion 140'. The former offers relatively higher image quality since the synchronisation signals and the transducer signals incur more similar electrical paths, although co-locating the clock circuit and the modulator circuit in the limited space available at the distal end of an IVUS imaging device incurs extra complexity.

Thus, in some examples, the clock circuit is disposed at a distal end of the interventional device, and the modulator circuit 180 is disposed in the device connector portion (140'). In these examples, the modulator circuit 180 receives the transducer signals and the synchronisation signals, which may be received as a combined signal if they represent detected IVUS imaging signals, and the modulator circuit 180 generates the electrical signals using the transducer signals and the synchronisation signals. The modulator circuit 180 is electrically coupled to the at least one transmitter 160 for transmitting the electrical signals using the at least one transmitter 160.

In other examples, the clock circuit is located in the device connector portion 140'. In these examples, the clock circuit is configured to generate the synchronization signals, and the clock circuit is electrically coupled to the at least one transmitter 160 for generating the electrical signals using the synchronization signals. The clock circuit may be used to synchronise a timing of electrical operations at the distal end 110" of the interventional device 110. For example, the clock circuit may be used to synchronise the generation of ultrasound signals by the interventional device. The clock circuit may be coupled to the transmitter 160, and to the distal end of the interventional device 110 separately, or the clock circuit may be coupled to the transmitter 160 via the distal end of the interventional device. Thus, in some examples, the device connector portion 140' is electrically coupled to the proximal end 110' of the interventional device 110; and the clock circuit is electrically coupled to the at least one transmitter 160 for generating the electrical signals using the synchronization signals, via the proximal end 110' of the interventional device 110, and via a distal end 110" of the interventional device 110, for further synchronising a timing of electrical operations at the distal end 110" of the interventional device 110; or the clock circuit is electrically coupled to the at least one transmitter 160 for generating the electrical signals using the synchronization signals, and to the distal end 110" of the interventional device 110 via the proximal end 110' of the interventional device 110, for further synchronising a timing of electrical operations at the distal end 110" of the interventional device 110.

The system described with reference to Fig. 4 may also include one or more features that were described above with reference to Fig. 3, Fig. 2 or Fig. 1. For example, a second set of electrical signals may be transmitted between the device connector portion 140' and the control unit connector portion 140". Additionally or alternatively, electrical power may be transmitted electromagnetically between the control unit connector portion 140" and the device connector portion 140'. Thus, as illustrated in Fig. 4, the connector may also include one or more of: a second transmitter 210, a second receiver 230, and the device connector portion 140' and the control unit connector portion 140" may each include a coil 220', 220''.

As mentioned above, the systems described with reference to the Figures may be used with various types of interventional device. In one example, the interventional device 110 comprises an intravascular ultrasound, IVUS, imaging device, wherein the synchronization signals comprise timing signals for generating ultrasound pulses with the IVUS imaging device, and wherein the transducer signals represent ultrasound reflectance measurements generated by the IVUS imaging device in response to the generated ultrasound pulses. In another example, the interventional device 110 comprises an intravascular ultrasound blood flow measurement device configured to determine blood flow measurements based on reflected ultrasound signals detected by the intravascular ultrasound blood flow measurement device in response to emitted ultrasound signals, and wherein the synchronization signals comprise timing signals for generating the emitted ultrasound pulses, and wherein the transducer signals represent the detected ultrasound signals. The systems may likewise be used with other types of interventional devices, such as an in-body tracking system, and so forth. In-body tracking systems are typically based on measurements of one or more of: electromagnetic, ultrasound, and dielectric impedance, tracking signals.

One or more processors may be configured to execute the functions disclosed in the invention. The physical location of the one or more processors is related to the structural component which is associated with the respective function. For instance when the control unit connector portion (140") is configured to extract the transducer signals and the synchronisation signals from the electrical signals, and to perform a timing correction on the transducer signals based on the synchronisation signals, then the processor that carries out these functions is physically located in the control unit connector portion.

The above examples are to be understood as illustrative of the present disclosure and not restrictive. Further examples are also contemplated. For instance, features described in relation to one of the example systems may be implemented in another of the example systems in a corresponding manner. It is therefore to be understood that a feature described in relation to any one example may be used alone, or in combination with other described features, and may also be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

### CLAUSES

Clause 1. A system for coupling electrical signals between an interventional device (110) and a control unit (120) through an electrically insulating barrier (130), the system comprising:
a connector (140), comprising a device connector portion (140') and a control unit connector portion (140");
wherein the device connector portion (140') is configured to be electrically coupled to a proximal portion (110') of the interventional device (110);
wherein the control unit connector portion (140") is configured to be electrically coupled to the control unit (120);
wherein the device connector portion (140'), and the control unit connector portion (140'') are configured to couple to each other across the electrically insulating barrier (130);
wherein the device connector portion (140') comprises at least one transmitter (160), configured to transmit electrical signals representing i) transducer signals and ii) synchronisation signals for the transducer signals, the transducer signals being generated at a distal portion (110") of the interventional device (110), and the synchronisation signals being generated at a distal portion (110") of the interventional device or in the device connector portion (140');
wherein the control unit connector portion (140") comprises at least one receiver (170) configured to receive the electrical signals when the control unit connector portion (140") and the device connector portion (140') are coupled to each other across the electrically insulating barrier (130); and
wherein the control unit connector portion (140") or the control unit (120), is configured to extract the transducer signals and the synchronisation signals from the electrical signals, and to perform a timing correction on the transducer signals based on the synchronisation signals.

Clause 2. The system according to clause 1, wherein the at least one transmitter (160) comprises a modulator circuit (180) configured to generate the electrical signals using the transducer signals and the synchronisation signals.

Clause 3. The system according to clause 1, further comprising the interventional device (110), a clock circuit configured to generate the synchronization signals, and a modulator circuit (180);
wherein the clock circuit is disposed at a distal portion of the interventional device, and the modulator circuit (180) is disposed in the device connector portion (140');
wherein the modulator circuit (180) receives the transducer signals and the synchronisation signals, and wherein the modulator circuit (180) is configured to generate the electrical signals using the transducer signals and the synchronisation signals; and
wherein the modulator circuit (180) is electrically coupled to the at least one transmitter (160) for transmitting the electrical signals using the at least one transmitter (160).

Clause 4. The system according to clause 2 or 3, wherein the modulator circuit (180) comprises a voltage controlled oscillator.

Clause 5. The system according to clause 1, wherein the device connector portion (140') comprises a clock circuit configured to generate the synchronization signals, and wherein the clock circuit is electrically coupled to the at least one transmitter (160) for generating the electrical signals using the synchronization signals.

Clause 6. The system according to clause 5, further comprising the interventional device (110), and wherein the device connector portion (110') is electrically coupled to the proximal portion (110') of the interventional device (110); and
wherein the clock circuit is electrically coupled to the at least one transmitter (160) for generating the electrical signals using the synchronization signals, via the proximal portion (110') of the interventional device (110), and via a distal portion (110") of the interventional device (110), for further synchronising a timing of electrical operations at the distal portion (110") of the interventional device (110); or
wherein the clock circuit is electrically coupled to the at least one transmitter (160) for generating the electrical signals using the synchronization signals, and to the distal portion (110") of the interventional device (110) via the proximal portion (110') of the interventional device (110), for further synchronising a timing of electrical operations at the distal portion (110") of the interventional device (110).

Clause 7. The system according to clause 1, wherein the control unit connector portion, or the control unit comprises a detector circuit (190) and a timing correction circuit (200);
wherein the detector circuit (190) is configured to extract the transducer signals and the synchronisation signals from the electrical signals; and
wherein the timing correction circuit (200) is configured to perform the timing correction on the transducer signals by using the synchronisation signals to adjust a timing of the transducer signals.

Clause 8. The system according to clause 1, wherein the electrical signals comprise a first bandwidth, and wherein the at least one transmitter (160, 210) is further configured to transmit a second set of electrical signals having a second bandwidth, the second bandwidth being smaller than the first bandwidth; and
wherein the second set of electrical signals represent i) a status of the interventional device (110) and/or ii) programming data for programming a processor disposed on the device connector portion (140') or on the interventional device (110) and/or iii) measurement data generated by a temperature sensor or an electrocardiography sensor or a power sensor disposed on the interventional device (110).

Clause 9. The system according to clause 1, wherein the device connector portion (140') and the control unit connector portion (140'') each include a coil (220', 220'') for transmitting electrical power electromagnetically between the control unit connector portion (140'') and the device connector portion (140').

Clause 10. The system according to clause 9, wherein device connector portion (140') further comprises at least one rectifier coupled to the coil (220') of the device connector portion (140') for rectifying electromagnetic signals received by the coil (220') from the coil (220'') of the control unit connector portion (140").

Clause 11. The system according to clause 1, wherein the interventional device (110) comprises an intravascular ultrasound, IVUS, imaging device, wherein the synchronization signals comprise timing signals for generating ultrasound pulses with the IVUS imaging device, and wherein the transducer signals represent ultrasound reflectance measurements generated by the IVUS imaging device in response to the generated ultrasound pulses; or
wherein the interventional device (110) comprises an intravascular ultrasound blood flow measurement device configured to determine blood flow measurements based on reflected ultrasound signals detected by the intravascular ultrasound blood flow measurement device in response to emitted ultrasound signals, and wherein the synchronization signals comprise timing signals for generating the emitted ultrasound pulses, and wherein the transducer signals represent the detected ultrasound signals.

Clause 12. The system according to clause 1, wherein the at least one transmitter (160) comprises at least one antenna for transmitting the electrical signals; and wherein at least one receiver (170) comprises at least one corresponding antenna for receiving the transmitted electrical signals.

Clause 13. The system according to clause 1, wherein the control unit connector portion (140'') and the device connector portion (140') are configured to couple to each other across the electrically insulating barrier (130) via magnetic force and/or via a mechanical fixture.

Clause 14. The system according to clause 1, wherein the device connector portion (140') and the control unit connector portion (140") are configured to couple to each other across the electrically insulating barrier (130) via a shape of the device connector portion (140') and a corresponding shape of the control unit connector portion (140").

Clause 15. A method of coupling electrical signals between an interventional device (110) and a control unit (120) through an electrically insulating barrier (130), the method comprising:
electrically coupling a device connector portion (140') of a connector (140) to a proximal portion (110') of the interventional device (110);
electrically coupling a control unit connector portion (140") of the connector (140) to the control unit (120);
coupling the device connector portion (140') and the control unit connector portion (140'') to each other across the electrically insulating barrier (130);
transmitting electrical signals from the device connector portion (140') and receiving the electrical signals at the control unit connector portion (140"), wherein the electrical signals represent i) transducer signals and ii) synchronisation signals for the transducer signals, the transducer signals being generated at a distal portion (110") of the interventional device (110), and the synchronisation signals being generated at a distal portion (110") of the interventional device or in the device connector portion (140');
extracting the transducer signals and the synchronisation signals from the electrical signals at the control unit connector portion (140") or at the control unit (120), and
performing a timing correction on the transducer signals based on the synchronisation signals.

## Claims

1. A device connector portion (140') of a connector (140) of a system for coupling electrical signals between an interventional device (110) and a control unit (120) through an electrically insulating barrier (130), wherein the device connector portion (140') is configured to:
be electrically coupled to a proximal portion (110') of the interventional device (110); and
couple to a control unit connector portion (140") of the connector (140) across the electrically insulating barrier (130),
wherein the device connector portion (140') comprises at least one transmitter (160), configured to transmit the electrical signals representing i) transducer signals and ii) synchronisation signals for the transducer signals, the transducer signals being generated at a distal portion (110") of the interventional device (110), and the synchronisation signals being generated at a distal portion (110") of the interventional device or in the device connector portion (140').

2. The device connector portion according to claim 1, wherein the at least one transmitter (160) comprises a modulator circuit (180) configured to generate the electrical signals using the transducer signals and the synchronisation signals.

3. The device connector portion of claim 1, having disposed therein a modulator circuit, wherein the modulator circuit (180) is configured to receive the transducer signals and the synchronisation signals, and wherein the modulator circuit (180) is configured to generate the electrical signals using the transducer signals and the synchronisation signals, and wherein the modulator circuit (180) is electrically coupled to the at least one transmitter (160) for transmitting the electrical signals using the at least one transmitter (160).

4. The device connector portion according to claim 2 or 3, wherein the modulator circuit (180) comprises a voltage controlled oscillator.

5. The device connector portion of any one of the claims 1 to 4, wherein the synchronization signals are generated by a clock circuit disposed at a distal portion of the interventional device.

6. The device connector portion of any one of claims 1 to 4, comprising a clock circuit configured to generate the synchronization signals, and wherein the clock circuit is electrically coupled to the at least one transmitter (160) for generating the electrical signals using the synchronization signals.

7. The device connector portion of claims 5 or 6 and the interventional device (110), wherein the device connector portion (110') is electrically coupled to the proximal portion (110') of the interventional device (110); and
wherein the clock circuit is electrically coupled to the at least one transmitter (160) for generating the electrical signals using the synchronization signals, via the proximal portion (110') of the interventional device (110), and via a distal portion (110") of the interventional device (110), for further synchronising a timing of electrical operations at the distal portion (110") of the interventional device (110); or
wherein the clock circuit is electrically coupled to the at least one transmitter (160) for generating the electrical signals using the synchronization signals, and to the distal portion (110") of the interventional device (110) via the proximal portion (110') of the interventional device (110), for further synchronising a timing of electrical operations at the distal portion (110") of the interventional device (110).

8. A control unit connector portion (140") of a connector of a system for coupling electrical signals between an interventional device (110) and a control unit (120) through an electrically insulating barrier (130),
wherein the control unit connector portion (140") is configured to:
be electrically coupled to the control unit (120); and
couple to a device connector portion (140') across the electrically insulating barrier (130);
wherein the control unit connector portion (140") comprises at least one receiver (170) configured to receive the electrical signals transmitted by the device connector portion when the control unit connector portion (140") is coupled to the device connector portion (140') across the electrically insulating barrier (130), the electrical signals representing i) transducer signals and ii) synchronisation signals for the transducer signals, the transducer signals being generated at a distal portion (110") of the interventional device (110), and the synchronisation signals being generated at a distal portion (110") of the interventional device or in the device connector portion (140'),
wherein the control unit connector portion (140") or the control unit (120), is configured to extract the transducer signals and the synchronisation signals from the electrical signals, and to perform a timing correction on the transducer signals based on the synchronisation signals.

9. The system according to claim 8, wherein the control unit connector portion, or the control unit comprises a detector circuit (190) and a timing correction circuit (200);
wherein the detector circuit (190) is configured to extract the transducer signals and the synchronisation signals from the electrical signals; and
wherein the timing correction circuit (200) is configured to perform the timing correction on the transducer signals by using the synchronisation signals to adjust a timing of the transducer signals.

10. The device connector portion of any one of the claims 1 to 7, or the control unit connector portion of any one of the claims 7 to 8, wherein the electrical signals comprise a first bandwidth, and wherein the at least one transmitter (160, 210) is further configured to transmit a second set of electrical signals having a second bandwidth, the second bandwidth being smaller than the first bandwidth; and
wherein the second set of electrical signals represent i) a status of the interventional device (110) and/or ii) programming data for programming a processor disposed on the device connector portion (140') or on the interventional device (110) and/or iii) measurement data generated by a temperature sensor or an electrocardiography sensor or a power sensor disposed on the interventional device (110).

11. The device connector portion (140') of any one of the previous claims, comprising a coil (220'), for transmitting electrical power electromagnetically between the control unit connector portion (140") and the device connector portion (140') using a coil of the control unit connector portion (140'').

12. The device connector portion of any one of the previous claims or the control unit connector portion of any one of the previous claims, wherein the interventional device (110) comprises an intravascular ultrasound, IVUS, imaging device, wherein the synchronization signals comprise timing signals for generating ultrasound pulses with the IVUS imaging device, and wherein the transducer signals represent ultrasound reflectance measurements generated by the IVUS imaging device in response to the generated ultrasound pulses; or
wherein the interventional device (110) comprises an intravascular ultrasound blood flow measurement device configured to determine blood flow measurements based on reflected ultrasound signals detected by the intravascular ultrasound blood flow measurement device in response to emitted ultrasound signals, and wherein the synchronization signals comprise timing signals for generating the emitted ultrasound pulses, and wherein the transducer signals represent the detected ultrasound signals.

13. The control unit connector portion (140") of any one of the previous claims or the device connector portion (140') of any one of the previous claims, further configured to couple to each other across the electrically insulating barrier (130) via magnetic force and/or via a mechanical fixture.

14. The control unit connector portion (140") of any one of the previous claims or the device connector portion (140') of any one of the previous claims, wherein the device connector portion (140') and the control unit connector portion (140'') are configured to couple to each other across the electrically insulating barrier (130) via a shape of the device connector portion (140') and a corresponding shape of the control unit connector portion (140").

15. A system for coupling electrical signals between an interventional device and a control unit through an electrically insulating barrier, the system comprising a connector, the connector comprising: a device connector portion of any one of claims 1 to 7 or of any one of claims 10 to 14; and a control unit connector portion of any one of claims 8, 9, or of any one of claims 10 to 13.
